(19) Europäisches Patentamt

European Patent Office

Office européen des brevets

(11) EP 0 528 256 B1

(12) **EUROPÄISCHE PATENTSCHRIFT**

(45) Veröffentlichungstag und Bekanntmachung des Hinweises auf die Patenterteilung:
**25.09.1996 Patentblatt 1996/39**

(51) Int Cl.$^6$: **C12P 41/00**, C07C 255/00, C12P 13/00

(21) Anmeldenummer: **92113262.7**

(22) Anmeldetag: **04.08.1992**

(54) **D-2,4-Dihydroxy-3,3-dimethylbutansäurenitril, seine Herstellung und Verwendung sowie Verfahren zur Herstellung von D-Pantolacton, D-2,4-Dihydroxy-3,3-dimethylbutansäureamid und D-Pantothensäure**

D-2,4-dihydroxy-3,3-dimethylbutyronitrile, its production and use and process for production of D-pantolactone, D-2,4-dihydroxy-3,3-dimethylbutyramide and D-pantothenic acid

D-2,4-dihydroxy-3,3-diméthylbutyronitrile, sa production et son utilisation et procédé de production de D-pantolactone, D-2,4-dihydroxy-3,3-diméthylbutyramide et D-acid pantothénique

(84) Benannte Vertragsstaaten:
**AT BE CH DE DK ES FR GB IT LI NL PT**

(30) Priorität: **12.08.1991 DE 4126580**

(43) Veröffentlichungstag der Anmeldung:
**24.02.1993 Patentblatt 1993/08**

(73) Patentinhaber: **DEGUSSA AG**
**D-60311 Frankfurt (DE)**

(72) Erfinder:
• **Beisswenger, Thomas, Dr.**
  **W-6368 Bad Vilbel-Gronau (DE)**
• **Huthmacher, Klaus, Dr.**
  **W-6460 Gelnhausen (DE)**
• **Klenk, Herbert, Dr.**
  **W-6450 Hanau 9 (DE)**

(56) Entgegenhaltungen:
EP-A- 0 172 371          EP-A- 0 326 063
DE-A- 3 823 866

• **PATENT ABSTRACTS OF JAPAN, unexamined applications, C Field, Band 13, Nr. 182, 27. April 1989 THE PATENT OFFICE JAPANESE GOVERNMENT Seite 84 C 591**
• **PATENT ABSTRACTS OF JAPAN, unexamined applications, C Field, Band 11, Nr. 163, 26. Mai 1987 THE PATENT OFFICE JAPANESE GOVERNMENT Seite 127 C 424**

**Beschreibung**

Die vorliegende Erfindung betrifft D-2,4-Dihydroxy-3,3-dimethylbutansäurenitril, das als chirale Zwischenstufe zur Herstellung von D-Pantolacton und D-Pantothensäure dient.

D-(-)Pantolacton ist ein wichtiges Zwischenprodukt bei der Synthese von D-(+)Pantothensäure oder deren Derivaten. D-(-)Pantolacton kann aus der racemischen Verbindung durch aufwendige Racematspaltungsverfahren in Form von Pantoinsäuresalzen mit chiralen Hilfsbasen und anschließender Spaltung der diastereomeren Salzpaare und Cyclisierung der Pantoinsäure zum Pantolacton erhalten werden (US-PS 3,884,966; DE 2404305; DE 2558508; US-PS 4,095,952). Das unerwünschte L-(+)Pantolacton muß, um einen wirtschaftlichen Prozeß zu erhalten, racemisiert werden und als DL-Pantolacton erneut zur Racematspaltung eingesetzt werden.

In anderen Verfahren (Tetrahedron Lett. (50), 4431-2, 1977; JP 53-105421 (1978); EP 218970; EP 320096) wird, durch enantioselektive katalytische Hydrierung, ausgehend vom Ketopantolacton, direkt das erwünschte D-(-)Pantolacton erhalten. Ketopantolacton ist aber schwer zugänglich bzw. wird in einer weiteren Reaktionsstufe aus DL-Pantolacton durch Oxidation hergestellt, so daß hier eine aufwendige Verfahrensweise vorliegt.

Aus JP 2-100692 (1990), JP 61-293386 (1986), JP 59-98695 (1984) und Angew. Chemie 100, 1988, 640 sind mikrobielle Verfahren bekannt, bei denen mit lebenden Bakterien- oder Pilzzellen eine enantioselektive Reduktion von Ketopantolacton erreicht wird. Auch hier muß das Ketopantolacton, wie oben beschrieben, durch Oxidation hergestellt werden.

Aus CA Vol. 111, 1989: 132610u ist ein Verfahren zur Herstellung des racemischen Pantolactons bekannt, bei dem das racemische 2,4-Dihydroxy-3,3-dimethylbutansäurenitril über drei Stufen enzymatisch umgesetzt wird. Das racemische Pantolacton muß, wie eingangs beschrieben, vor der Umsetzung zur Pantothensäure in die einzelnen Enantiomere aufgetrennt werden.

W. Becker und E. Pfeil beschreiben in Biochemische Zeitschrift 346, 301 - 321 (1966) verschiedene Reaktionen der D-Oxynitrilase, wobei u. a. kinetische Studien durchgeführt wurden. Das Enzym bildet aus einer Vielzahl von Aldehyden rechtsdrehende, linksdrehende oder racemische α-Hydroxynitrile, wobei im Normalfall auf 10 mmol Aldehyd 1 mmol Enzym eingesetzt wird. Bei zunächst nicht umgesetzten Aldehyden kann die Enzymmenge auf 5 mg erhöht werden.

Aus der DE-OS 38 23 866 ist ein Verfahren zur Herstellung von S-Cyanhydrinen durch Umsetzung der entsprechenden Oxoverbindungen und Blausäure mit Hilfe von Oxynitrilase (4.1.2.11) aus Sorghum bicolor bekannt. Mit Hilfe dieser Oxynitrilase lassen sich die entsprechenden optischen Antipoden zur Umsetzung mit Oxynitrilase 4.1.2.10 darstellen.

In EP-A-0 326 063 ist ein Verfahren zur Herstellung optisch aktiver (R)-Cyanhydrine durch Umsetzung von Aldehyden oder Ketonen mit Blausäure in wäßriger Lösung in Gegenwart von (R)-Oxynitrilase beschrieben. Das Verfahren eignet sich zur Umsetzung von z. B. Pivalinaldehyd unter Verwendung geringer Enzymkonzentrationen.

Aufgabe der vorliegenden Erfindung ist die Bereitstellung eines für die Synthese von D-(-)Pantolacton geeigneten Zwischenproduktes, das einfach und kostengünstig erhältlich sein soll und aus dem das D-(-)Pantolacton bzw. 0-Pantothensäure auf einfache Weise in akzeptabler optischer Reinheit erhältlich sein soll.

Diese Aufgabe wird gelöst durch 0-2,4-Dihydroxy-3,3-dimethylbutansäurenitril, das vorteilhaft eine optische Reinheit (D:L) von mindestens 80:20 haben soll. Vorzugsweise hat das Nitril eine höhere optische Reinheit als 85:15, besonders bevorzugt 90:10 und darüber. Durch Verseifung des Nitrils mit konzentrierten Säuren läßt sich in hohen Ausbeuten das D-(-)Pantolacton darstellen, wobei die optische Reinheit weitgehend erhalten bleibt. Das bei der Verseifung in geringem Umfang aus D-Nitril (bis ca. 5 %) und aus eventuell vorhandenem L-Nitril entstehende L-Pantolacton kann durch die bekannten Verfahren, z.B. durch Kristallisation, abgetrennt werden.

Das erfindungsgemäße Nitril ist erhältlich durch Umsetzung von Hydroxypivalaldehyd mit Cyanwasserstoff in Gegenwart einer D-Oxynitrilase. Eine geeignete D-Oxynitrilase ist Oxynitrilase EC 4.1.2.10. Die Umsetzung wird in einem wässrigen Medium durchgeführt, wobei die D-Oxynitrilase in einer Konzentration von mindestens 20 mg bzw. 2000 U pro mmol Hydroxypivalaldehyd eingesetzt wird. 1 mg der hier verwendeten D-Oxynitrilase entspricht ca. 100 Units, wobei 1 Unit des Enzyms 1,0 µmol Mandelsäurenitril zu Benzaldehyd und Cyanwasserstoff bei einem pH von 5,4 und 25°C pro Minute umsetzt.

Der Hydroxypivalaldehyd wird üblicherweise in einer Konzentration von 10 - 200 mmolar, vorzugsweise 20 - 50 mmolar eingesetzt. Außerhalb dieser Grenzen sinkt die Enantiomerenausbeute ab. Der Cyanwasserstoff wird vorzugsweise in einer zum Hydroxypivalaldehyd mindestens äquimolaren Menge eingesetzt, üblicherweise wird mit einem Cyanwasserstoffüberschuß von bis zu 30 eq verfahren. Vorteilhaft ist die Verwendung von 2 - 5 Moläquivalenten Cyanwasserstoff bezogen auf Hydroxypivalaldehyd. Der Cyanwasserstoff kann dem Reaktionsansatz entweder als Reinsubstanz oder in wässriger Lösung oder in Pufferlösungen zugesetzt werden.

Als Reaktionsansatz wird im allgemeinen eine wässrige Pufferlösung, vorzugsweise ein Citronensäurepuffer verwendet. Die Konzentration des Puffers beträgt üblicherweise 0,01 - 0,5 molar, vorzugsweise 0,03 - 0,2 molar, wobei der pH-Wert der Pufferlösung durch Alkali- oder Erdalkalihydroxid oder durch Ammonium-Verbindungen eingestellt wird. Andere verwendbare Puffer sind z.B. Acetat-, Phosphat-, Borat-, Phthalat-Puffer oder Gemische dieser Pufferlösungen.

Die Reaktion wird vorzugsweise bei Temperaturen durchgeführt, bei denen das Enzym eine hohe Aktivität aufweist. Dies ist insbesondere der Temperaturbereich von 0 - 50°C, vorteilhaft sind 20 - 30°C. Der pH der Pufferlösung kann zwischen 2,8 - 8,0 liegen, bevorzugt sind leicht saure Reaktionslösungen mit einem pH von 2,8 - 5,2, besonders vorteilhaft sind 3,0 - 4,4. Ist der pH zu sauer, dann wird das Enzym zu schnell desaktiviert, ist der pH zu alkalisch, treten chemische Nebenreaktionen auf, die unter anderem zur Bildung des unerwünschten L-Enantiomeren führen.

Die Synthese optisch aktiver Cyanhydrine wurde von Becker und Pfeil, Biochemische Zeitschrift 346 (1966) 301 beschrieben. Nach der dort gegebenen Vorschrift ließen sich jedoch zahlreiche aliphatische gesättigte Aldehyde nicht zu optisch aktiven Produkten umsetzten. Aus der DE-OS 3802642 ist ein analoges Verfahren zur Herstellung optisch aktiver Cyanhydrine bekannt. Mit den dort gegebenen Reaktionsbedingungen konnten Acetaldehyd, Benzaldehyd und Furfurol zu den optisch aktiven Cyanhydrinen umgesetzt werden. Wird unter diesen Reaktionsbedingungen jedoch Hydroxypivalaldehyd umgesetzt, so kann keine optische Aktivität der Lösung bzw. kein Enantiomerüberschuß an gebildetem D-2,4-Dihydroxy-3,3-dimethylbutansäurenitril nachgewiesen werden.

Überraschenderweise wurde jedoch festgestellt, daß bei der Anwendung stark erhöhter Enzymkonzentrationen von mindestens 20 mg pro Mol Hydroxypivalaldehyd doch D-2,4-Dihydroxy-3,3-dimethylbutansäurenitril bevorzugt gebildet wird und unter geeigneten Bedingungen sehr hohe Enantiomerüberschüsse erzielbar sind. Gemäß dem Stand der Technik wurden bei den anderen Verbindungen nur 0,1 - 0,5 mg Enzym je mmol Substrat eingesetzt.

Vorzugsweise werden 50 mg Enzym pro mmol Hydroxypivalaldehyd oder mehr eingesetzt, vorteilhaft ist eine Menge von 80 mg/mmol und mehr. Wird zu wenig Enzym eingesetzt, dann kann kein oder kein genügend hoher Enatiomerenüberschuß erhalten werden, die Obergrenze der Enzymmenge wird durch wirtschaftliche Faktoren (Kosten, Reinigung etc.) bestimmt.

Zur Isolierung und Reinigung des D-2,4-Dihydroxy-3,3-dimethylbutansäurenitrils (Cyanhydrin) wird die Reaktionslösung nach einer Reaktionszeit von üblicherweise 30 Minuten bis zu 24 Stunden mit einem mit Wasser nicht vollständig mischbaren organischen Lösungsmittel, z.B. Essigsäureester, Dialkylether, Ketonen wie Isobutylmethylketon, oder Chlorkohlenwasserstoffen extrahiert. Zur Stabilisierung des Cyanhydrins wird dann zum organischen Extrakt eine kleine Menge an Salzsäure zugesetzt (ca. 10 - 50 µmol/mmol Cyanhydrin). Die organischen Extrakte werden vereinigt und im Vakuum eingeengt. Die Lösungsmittelreste können dabei durch Behandeln im Feinvakuum nahezu vollständig entfernt werden. Das so erhaltene Cyanhydrin kann nach Derivatisierung auf seine Enantiomerenzusammensetzung untersucht werden.

Überraschenderweise kann das so gebildete D-2,4-Dihydroxy-3,3-dimethylbutansäurennitril unter weitgehendem Erhalt der Chiralität in hohen Enantiomerüberschüssen zum D-2,4-Dihydroxy-3,3-dimethylbutansäureamid bzw. D-Pantolacton verseift werden, letzteres dient als Zwischenverbindung für das Vitamin Pantothensäure oder deren Derivate, z.B. Panthenol. Hierzu wird der Cyanhydrin-Extrakt mit wenig Wasser aufgenommen und in konzentrierte Mineralsäure eingegossen. In einfacher Art und Weise kann aber auch unter Verzicht einer Isolierung des Cyanhydrins direkt der gesamte Reaktionsansatz in die Mineralsaure eingegossen werden. Unter milden Bedingungen erhält man das Amid, das jedoch meist sofort zum D-Pantolacton weiter umgesetzt wird.

Zur Erfindung gehört daher auch ein Verfahren zur Herstellung von D-2,4-Dihydroxy-3,3-dimethylbutansäureamid, ein Verfahren zur Herstellung von D-Pantolacton sowie ein Verfahren zur Herstellung von D-Pantothensäure, ihren Salzen oder Derivaten. Die Verfahren zur Herstellung des Amids bzw. des D-Pantolactons zeichnen sich dadurch aus, daß D-2,4-Dihydroxy-3,3-dimethylbutansäurenitril verseift wird. Die Verseifung erfolgt vorteilhaft in einem wässrigen Medium und insbesondere in einem sauren Milieu. Unter alkalischen Bedingungen besteht die Gefahr der Zersetzung des Nitrils. Das Verfahren zur Herstellung von D-Pantothensäure zeichnet sich dadurch aus, daß D-2,4-Dihydroxy-3,3-dimethylbutansäurenitril zum D-Pantolacton verseift wird, das anschließend mit β-Alanin zur D-Pantothensäure weiter umgesetzt wird.

Die Umsetzung des Cyanhydrins zum D-Pantolacton wird vorteilhaft bei einer erhöhten Temperatur von ca. 50°C bis zur Siedehitze durchgeführt. Als Mineralsäure kommt beispielsweise Schwefelsäure oder konzentrierte Salzsäurelösung in Betracht. Nach einer Reaktionszeit von üblicherweise 1 bis 5 Stunden kann die Lösung mit einem geeigneten organischen Lösungsmittel, das nicht vollständig mit Wasser mischbar sein soll, extrahiert werden. Hierfür können beispielsweise Essigsäureester, Dialkylether, aliphatische oder aromatische Kohlenwasserstoffe, Ketone wie Isobutylmethyketon oder Halogenkohlenwasserstoffe verwendet werden. Die vereinigten organischen Phasen werden im Vakuum eingeengt, im Rückstand ist das optisch aktive D-Pantolacton als Rohprodukt. Das D-Pantolacton kann mit β-Alanin zur D-Pantothensäure in wasserfreiem Lösungsmittel, z.B. Methanol, umgesetzt werden (JACS, 62, (1940) 1785). D-(+) Panthenol wird entsprechend mit 2-Aminoethanol dargestellt.

Zur Herstellung, z. B. des kristallinen Calcium-Salzes der Pantothensäure, wird beispielsweise das kristalline Calcium-Salz des β-Alanins in wasserfreiem Lösungsmittel aufgenommen und mit einer wasserfreien Lösung von D-Pantolacton versetzt. Die Reaktionsmischung wird einige h bei erhöhter Temperatur (40 - 65°C) gerührt und dann zur Kristallisation auf 0 - 30°C abgekühlt. Nach Trocknung erhält man kristallines D-Calciumpantothenat.

Nachfolgend wird die Erfindung anhand von Beispielen näher ausgeführt.

Beispiel 1

203 mg (2 mmol) Hydroxypivalaldehyd wurden in 40 ml Citratpuffer (0,1 molar; pH 3,3) gelöst und mit 20 ml Oxynitrilaselösung (ca. 10 mg/ml aus Prunus amygdalus) versetzt. Anschließend wurden 110 mg (4 mmol) Blausäurelösung (5,29 molar in $H_2O$) zugegeben. Nach 4 Stunden wurde die Reaktionslösung viermal mit 30 ml Isobutylmethylketon extrahiert und zur Stabilisierung mit 10 µl konzentrierter Salzsäure versetzt. Die organischen Phasen wurden vereinigt und im Vakuum eingeengt. Man erhielt 250 mg (96 % d Th.) 2,4-Dihydroxy-3,3-dimethylbutansäurenitril.

Die Bestimmung der optischen Reinheit erfolgte als O,O'-Bistrifluoracetyl-2,4-dihydroxy-3,3-dimethylbutansäurenitril kapillargaschromatographisch nach H. Frank et al (J. Chrom. 146, 1987, 197-206) auf einer chiralen Trennphase (FC-Chirasil-Val 25 m x 0,32 mm).

Die Derivatisierung wurde wie folgt vorgenommen: 3,5 mg 2,4-Dihydroxy-3,3-dimethylbutansäurenitril wurden in 0,2 ml Methylenchlorid gelöst und mit 0,2 ml Trifluoracetanhydrid versetzt. Anschließend wurde 10 min auf 110°C erhitzt. Nach dem Abkühlen wurde das Methylenchlorid mit Stickstoff abgedampft und der Rückstand in 0,15 ml Diethylketon gelöst.

Man erhielt 2 Peaks mit einem Flächenverhältnis von 90.7 % : 9,3 %, wobei das D-0,0'-Bistrifluoracetyl-2,4-dihydroxy-3,3-dimethylbutansäurenitril früher eluierte.

Beispiel 2:

Ein analoger Ansatz wie im Beispiel 1 wurde nach einer Reaktionszeit von 4 Stunden in 10 ml konzentrierte Schwefelsäure gegossen und 1 Stunde bei 100°C gerührt. Anschließend wurde auf ca. 30 ml eingeengt und viermal mit 30 ml Isobutylmethylketon extrahiert. Die vereinigten organischen Phasen wurden im Vakuum eingeengt und ergaben 210 mg (81 % der Theorie), 95 % Reinheit Pantolacton $[\alpha]_D^{20}$:-34,2° (c= 1, $H_2O$).

Die Bestimmung des Enantiomerüberschusses erfolgte als O-Trifluoracetylpantolacton, die Derivatisierung wurde, wie in Beispiel 1 aufgezeigt, vorgenommen. Die Analyse wurde wie in Beispiel 1 kapillargaschromatographisch durchgeführt.

Man erhielt 2 Peaks mit einem Flächenverhältnis von 15,0 % : 85,0 %, wobei das D-O-Trifluoracetylpantolacton später eluierte.

Beispiel 3

Ein analoger Ansatz wie in Beispiel 1 wurde nach einer Reaktionszeit von 2 Stunden in 10 ml siedende konzentrierte Salzsäure gegossen und 1 Stunde bei 100°C gerührt. Anschließend wurde auf ca. 30 ml eingeengt und viermal mit 30 ml Isobutylmethylketon extrahiert. Die vereinigten organischen Phasen wurden im Vakuum eingeengt und ergaben 160 mg (62 % der Theorie) an Pantolacton (98 % Reinheit) $[\alpha]_D^{20}$:-36.0° (c=1, $H_2O$).

Die Derivatisierung und Bestimmung des Enantiomerüberschusses wurde wie in Beispiel 2 durchgeführt.

Man erhielt 2 Peaks mit einem Flächenverhältnis von 14,4 % : 85,6 %, wobei das D-O-Trifluoracetylpantolacton später eluierte.

Beispiel 4

203 mg (2,0 mmol) Hydroxypivalaldehyd wurden in 36 ml Citratpuffer (0.1 molar; pH = 3,0) gelöst und mit 22,5 ml Oxynitrilaselösung (10 mg/ml aus Prunus amygdalus) versetzt. Anschließend wurden 1,22 g (45 mmol) Cyanwasserstoff zugegeben und bei Raumtemperatur gerührt. Nach 60 min wurde die Reaktionslösung mit viermal je 30 ml Isobutylmethylketon extrahiert und die vereinigten organischen Phasen wurden zur Stabilisierung mit 10 µl konzentrierter Salzsäure versetzt. Die organischen Phasen wurden im Vakuum eingeengt. Man erhielt 175 mg (67 % der Theorie) 2,4-Dihydroxy-3,3-dimethylbutansäurenitril.

Die Bestimmung der Enantiomeranteile wurde wie in Beispiel 1 beschrieben durchgeführt. Man erhielt 2 Peaks mit einem Flächenverhältnis von 83,7:16,3 wobei das D-O,O'-Bistrifluoracetyl-2,4-dihydroxy-3,3-dimethylbutansäurenitril früher eluierte.

Beispiel 5

203 mg (2,0 mmol) Hydroxypivalaldehyd wurden in 50 ml Citratpuffer (0,1 molar; pH = 4,0) gelöst und mit 10 ml Oxynitrilaselösung (ca. 10 mg/ml) versetzt. Anschließend wurden 110 mg (4.0 mmol) Cyanwasserstoff zugegeben und bei Raumtemperatur 60 min gerührt. Die Aufarbeitung analog Beispiel 4 ergab 180 mg (69 % der Theorie) 2,4-Dihydroxy-3,3-dimethylbutansäurenitril mit einem Enantiomerverhältnis von 80,5:19,5.

Beispiel 6

0,32 g kristallines Calcium-β-alaninat werden in 2 ml Methanol aufgenommen und mit 0,39 g D-Pantolacton in 1 ml Methanol versetzt. Die Reaktionsmischung wird bei 60°C für 4 h gerührt und dann auf 5°C abgekühlt. Man erhält nach Trocknung des isolierten Feststoffs 0,54 g kristallines D-Calcium-pantothenat.

**Patentansprüche**

1. D-2,4-Dihydroxy-3,3-dimethylbutansäurenitril.

2. D-2,4-Dihydroxy-3,3-dimethylbutansäurenitril mit optischer Reinheit (D:L) von mindestens 80:20.

3. Nitril nach Anspruch 2,
   **gekennzeichnet durch**
   eine optische Reinheit (D:L) von mindestens 85:15, vorzugsweise 90:10.

4. Verfahren zur Herstellung von D-2,4-Dihydroxy-3,3-dimethylbutansäurenitril,
   **dadurch gekennzeichnet,**
   daß man Hydroxypivalaldehyd mit Cyanwasserstoff in einem wässrigen Medium in Gegenwart von mindestens 20 mg D-Oxynitrilase je mmol Hydroxypivalaldehyd umsetzt.

5. Verfahren zur Herstellung von D-2,4-Dihydroxy-3,3-dimethylbutansäurenitril,
   **dadurch gekennzeichnet,**
   daß man Hydroxypivalaldehyd mit Cyanwasserstoff in einem wässrigen Medium in Gegenwart von mindestens 2000 U D-Oxynitrilase je mmol Hydroxypivalaldehyd umsetzt.

6. Verfahren nach Anspruch 4 oder 5,
   **dadurch gekennzeichnet,**
   daß man in einem wässrigen Puffersystem bei pH 2,8 bis 8,0 umsetzt.

7. Verfahren nach Anspruch 6,
   **dadurch gekennzeichnet,**
   daß man bei einer Pufferkonzentration von 0,01 bis 0,5 molar umsetzt.

8. Verfahren nach Anspruch 6 oder 7,
   **dadurch gekennzeichnet,**
   daß als Puffer ein Citrat-, Borat-, Acetat-, Phosphat-, Phthalat-Puffer oder ein Gemisch verwendet wird.

9. Verfahren nach einem der Ansprüche 4 bis 8,
   **dadurch gekennzeichnet,**
   daß man bei einer Temperatur von 0 bis 50°C umsetzt.

10. Verfahren zur Herstellung von D-2,4-Dihydroxy-3,3-dimethylbutansäureamid oder D-Pantolacton,
    **dadurch gekennzeichnet,**
    daß man D-2,4-Dihydroxy-3,3-dimethylbutansäurenitril verseift.

11. Verfahren nach Anspruch 10,
    **dadurch gekennzeichnet,**
    daß man im sauren Milieu verseift.

12. Verfahren zur Herstellung von D-Pantothensäure oder ihrer Salze,
    **dadurch gekennzeichnet,**
    daß man D-2,4-Dihydroxy-3,3-dimethylbutansäurenitril verseift und das so hergestellte D-Pantolacton mit β-Alanin zur D-Pantothensäure oder ihren Salzen umgesetzt wird.

13. Verfahren zur Herstellung von D-Panthenol,
    **dadurch gekennzeichnet,**
    daß man D-2,4-Dihydroxy-3,3-dimethylbutansäurenitril verseift und das so hergestellte D-Pantolacton mit 2-Aminoethanol zum D-Panthenol umgesetzt wird.

14. Verwendung des Nitrils nach einem der Ansprüche 1 bis 3 zur Synthese von D-Pantolacton.

15. Verwendung des Nitrils nach einem der Ansprüche 1 bis 3 zur Synthese von D-Pantothensäure, ihrer Salze oder ihrer Derivate.

**Claims**

1. D-2,4-dihydroxy-3,3-dimethylbutanonitrile.

2. D-2,4-dihydroxy-3,3-dimethylbutanonitrile with an optical purity (D:L) of at least 80:20.

3. A nitrile according to Claim 2,
   characterised by an optical purity (D:L) of at least 85:15, preferably 90:10.

4. A process for preparing D-2,4-dihydroxy-3,3-dimethylbutanonitrile, characterised in that hydroxypivalaldehyde is reacted with hydrogen cyanide in an aqueous medium in the presence of at least 20 mg of D-oxynitrilase per mmol of hydroxypivalaldehyde.

5. A process for preparing D-2,4-dihydroxy-3,3-dimethyl-butanonitrile,
   characterised in that hydroxypivalaldehyde is reacted with hydrogen cyanide in an aqueous medium in the presence of at least 2000 U of D-oxynitrilase per mmol of hydroxypivalaldehyde.

6. A process according to Claim 4 or 5,
   characterised in that the reaction is performed in an aqueous buffer system at pH 2.8 to 8.0.

7. A process according to Claim 6,
   characterised in that the reaction is performed at a buffer concentration of 0.01 to 0.5 molar.

8. A process according to Claim 6 or 7,
   characterised in that a citrate, borate, acetate, phosphate or phthalate buffer or a mixture is used as buffer.

9. A process according to one of Claims 4 to 8, characterised in that the reaction is performed at a temperature of 0 to 50°C.

10. A process for preparing D-2,4-dihydroxy-3,3-dimethyl-butanoamide or D-pantolactone, characterised in that D-2,4-dihydroxy-3,3-dimethyl-butanonitrile is saponified.

11. A process according to Claim 10, characterised in that saponification is performed in an acid environment.

12. A process for preparing D-pantothenic acid or its salts, characterised in that D-2,4-dihydroxy-3,3-dimethyl-butanonitrile is saponified and the D-pantolactone so prepared is reacted with β-alanine to give D-pantothenic acid or its salts.

13. A process for preparing D-panthenol, characterised in that D-2,4-dihydroxy-3,3-dimethyl-butanonitrile is saponified and the D-pantolactone so prepared is reacted with 2-aminoethanol to give D-panthenol.

14. Use of the nitrile according to one of Claims 1 to 3 to synthesise D-pantolactone.

15. Use of the nitrile according to one of Claims 1 to 3 to synthesise D-pantothenic acid, its salts or its derivatives.


**Revendications**

1. Nitrile D-2,4-dihydroxy-3,3-diméthylbutanoïque.

2. Nitrile D-2,4-dihydroxy-3,3-diméthylbutanoïque avec une pureté optique (D:L) d'au moins 80:20.

3. Nitrile selon la revendication 2, caractérisé par une pureté optique (D:L) d'au moins 85:15, de préférence 90:10.

4. Procédé d'obtention du nitrile D-2,4-dihydroxy-3,3-diméthylbutanoïque, caractérisé en ce que l'on fait réagir l'hydroxypivalaldéhyde avec de l'acide cyanhydrique en milieu aqueux en présence d'au moins 20 mg de D-oxynitrilase par mmol d'hydroxypivalaldéhyde.

5. Procédé d'obtention du nitrile D-2,4-dihydroxy-3,3-diméthylbutanoïque, caractérisé en ce que l'on fait réagir l'hydroxypivalaldéhyde avec l'acide cyanhydrique en milieu aqueux en présence d'au moins 2000 U de D-oxynitrilase par mmol d'hydroxypivalaldéhyde.

6. Procédé selon la revendication 4 ou la revendication 5, caractérisé en ce que l'on fait réagir dans un système aqueux tampon à un pH de 2,8 à 8,0.

7. Procédé selon la revendication 6, caractérisé en ce que l'on fait réagir à une concentration de tampon de 0,01 à 0,5 molaire.

8. Procédé selon la revendication 6 ou la revendication 7, caractérisé en ce que l'on utilise, comme tampon, un tampon de citrate, borate, acétate, phosphate, phtalate ou un mélange de tampons.

9. Procédé selon l'une des revendications 4 à 8, caractérisé en ce que l'on fait réagir à une température allant de 0 à 50°C.

10. Procédé d'obtention de l'amide D-2,4-dihydroxy-3,3-diméthylbutanoïque ou de la D-pantolactone, caractérisé en ce que l'on saponifie le nitrile D-2,4-dihydroxy-3,3-diméthylbutanoïque.

11. Procédé selon la revendication 10, caractérisé en ce que l'on saponifie en milieu acide.

12. Procédé d'obtention d'acide D-pantothénique ou de ses sels, caractérisé en ce que l'on saponifie le nitrile D-2,4-dihydroxy-3,3-diméthylbutanoïque et en ce que l'on fait réagir la D-pantolactone ainsi préparée avec la β-alanine en acide D-pantothénique ou en ses sels.

13. Procédé d'obtention de D--panthénol caractérisé en ce que l'on saponifie le nitrile D-2,4-dihydroxy-3,3-diméthylbutanoïque et que l'on fait réagir la D-pantolactone ainsi produite avec le 2-aminoéthanol, en D-panthénol.

14. Utilisation du nitrile selon l'une des revendications 1 à 3, en vue de la synthèse de la D-pantolactone.

**15.** Utilisation du nitrile selon l'une des revendications 1 à 3, en vue de la synthèse de l'acide D-pantothénique, de ses sels ou de ses dérivés.